(19) Europäisches Patentamt
European Patent Office
Office européen des brevets

(11) Veröffentlichungsnummer: **0 263 953 B1**

(12) # EUROPÄISCHE PATENTSCHRIFT

(45) Veröffentlichungstag der Patentschrift: **31.07.91**

(51) Int. Cl.[5]: **C07F 15/00**, B01J 31/40, //B01D61/00

(21) Anmeldenummer: **87112611.6**

(22) Anmeldetag: **29.08.87**

(54) **Verfahren zur Abtrennung von Rhodiumkomplexverbindungen aus wässrigen Lösungen.**

(30) Priorität: **09.09.86 DE 3630587**

(43) Veröffentlichungstag der Anmeldung: **20.04.88 Patentblatt 88/16**

(45) Bekanntmachung des Hinweises auf die Patenterteilung: **31.07.91 Patentblatt 91/31**

(84) Benannte Vertragsstaaten: **AT BE DE FR GB IT SE**

(56) Entgegenhaltungen:
**DE-A- 1 912 380**
**GB-A- 1 312 076**
**US-A- 4 363 765**

**CHEMICAL ABSTRACTS, Band 99, Nr. 4, 25. Juli 1983, Seite 208, Spalte 1, Zusammenfassungsnr. 25923k, Columbus, Ohio, US; TEIJIN LTD.: "Recovery of metals from spent coating solutions of low pH", & JP - A - 57 207 130 (KOKAI TOKKYO KOHO) 18.12.1982**

(73) Patentinhaber: **HOECHST AKTIENGESELLSCHAFT**
**Postfach 80 03 20**
**W-6230 Frankfurt am Main 80(DE)**

(72) Erfinder: **Greb, Wolfgang, Dr., Dipl.-Ing.**
**Südstrasse 181**
**W-4220 Dinslaken(DE)**
Erfinder: **Hibbel, Josef, Dipl.-Ing.**
**Bruchsteg 13**
**W-4200 Oberhausen 11(DE)**
Erfinder: **Much, Joachim, Dipl.-Ing.**
**Im Torfveen 2**
**W-4200 Oberhausen 11(DE)**
Erfinder: **Schmidt, Volkmar, Dipl.-Ing.**
**Lützowstrasse 51**
**W-4200 Oberhausen 11(DE)**

EP 0 263 953 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Trennung in wäßriger Lösung enthaltener Katalytisch aktiver Rhodiumkomplexverbindungen einerseits, von in derselben Lösung vorliegenden überschüssigen, d.h. ungebundenen Komplexliganden, deren Umwandlungs- und Abbauprodukte sowie Verunreinigungen andererseits.

Rhodiumkomplexverbindungen werden zusammen mit überschüssigem Liganden in Form wäßriger Lösungen als Katalysatoren in verschiedenen Verfahren eingesetzt.

In der DE-PS 26 27 354 ist ein Hydroformylierungsverfahren beschrieben, das solche Katalysatoren verwendet. Sie werden unter den Reaktionsbedingungen aus Rhodium, das in metallischer Form oder als Verbindung eingesetzt wird und wasserlöslichem, organischem Phosphin, das im Überschuß vorliegt, gebildet. Die Wasserlöslichkeit der Phosphin-Liganden ist auf die Anwesenheit von Sulfonsäuregruppen im Molekül zurückzuführen. Die Phosphine gelangen bevorzugt in Form der Alkali-, Ammonium- oder Erdalkalisulfonate zum Einsatz. Dieses Verfahren zeichnet sich insbesondere durch hohe Selektivität hinsichtlich der Bildung geradkettiger Aldehyde aus. Überdies vermeidet es die Entstehung größerer Mengen hochsiedender Produkte.

Im Laufe der Zeit nimmt bei kontinuierlicher Arbeitsweise oder bei wiederholtem Einsatz derselben Katalysatorlösung die Wirksamkeit des Katalysatorsystems ab, sehr selektiv geradkettige Aldehyde zu bilden. Dieser Selektivitätsverlust hat verschiedene Ursachen. Zu ihnen gehören Ka talysatorgifte wie Eisencarbonyl, das sich durch Einwirkung von Synthesegas auf die Synthesegastransportleitungen oder das Konstruktionsmaterial des Reaktors bildet, und höhersiedende Kondensationsprodukte, die aus den Aldehyden entstehen. Selektivitätsmindernd wirkt auch die Abnahme des Verhältnisses von Phosphin zu Rhodium, das im neu eingesetzten Katalysator 50 bis 100 Mole Phosphin je 1 g-Atom Rhodium beträgt. Die Änderung des Phosphin-Rhodium-Verhältnisses bei längerer Anwendung des Katalysatorsystems ist eine Folge von Abbau- und Oxidationsprozessen, denen die sulfonierten Phosphine unterworfen sind. Im Verlauf dieser Reaktionen werden z.B. Phosphinoxide, Phosphinsulfide, aromatische Sulfonsäuren und Disulfophenylphosphinsäure, jeweils in Form ihrer Salze, gebildet.

Weder Phosphinoxide und Phosphinsulfide, noch die Salze aromatischer Sulfonsäuren und der Disulfophenylphosphinsäure sind allein oder zusammen mit Rhodium katalytisch wirksam.

Es ist daher zweckmäßig, die mit Spaltprodukten beladene, wäßrige Katalysatorlösung von Zeit zu Zeit insgesamt oder teilweise durch frische Lösung zu ersetzen. Die gebrauchte Katalysatorlösung enthält neben den obengenannten Umwandlungs- und Abbauprodukten der sulfonierten Phosphine Rhodium als Komplexverbindung sowie überschüssiges sulfoniertes Phosphin in Form wasserlöslicher Salze und häufig auch Verunreinigungen, die mit den Reaktanten eingeschleppt werden. Um die Wirtschaftlichkeit des Verfahrens zu sichern, ist es erwünscht, sowohl den Rhodiumkomplex als auch überschüssiges, aktives Phosphin zurückzugewinnen.

In der DE 32 35 029 wird ein Verfahren zur Rückgewinnung von Katalysatorsystemen, die wasserlösliches Rhodium, sulfonierte organische Phosphine und Kationen enthalten, beschrieben. Hierbei setzt man der wäßrigen Lösung des Katalysatorsystems zunächst eine den vorhandenen Säuregruppen mindestens äquivalente Menge Säure zu. Darauf wird mit einem Amin, das in einem organischen Lösungsmittel gelöst ist, extrahiert und die abgetrennte organische Phase, sie enthält das Aminsalz des sulfonierten Phosphins, mit der wäßrigen Lösung einer anorganischen Base, wie NaOH, innig vermischt. Es bilden sich zwei Phasen, eine wäßrige, die das Phosphinsulfonat und die Rhodiumkomplexverbindung enthält und eine organische, in der das Amin vorliegt. Die wäßrige Lösung kann unmittelbar oder nach Verdünnung mit Wasser oder nach Zusatz von sulfoniertem Phosphin wieder als Katalysatorlösung eingesetzt werden.

Dieses Verfahren liefert stets ein Gemisch aus der Rhodiumkomplexverbindung und dem überschüssigen Liganden. Eine gezielte Trennung von Rhodiumkomplexverbindung und den anderen Komponenten ist nur in begrenztem Maße möglich.

Die DE-A-19 12 380 betrifft ein Verfahren zur Abtrennung von Koordinationskomplexen von Übergangsmetallen aus einem homogenen fließfähigen Gemisch der Komplexe mit einer oder mehreren organischen Komponenten unter Verwendung von Cellulosemembranen. Diese Arbeitsweise wird u.a. auch zur Abtrennung von Rhodiumkomplexverbindungen aus Gemischen verwendet, die die Umsetzungsprodukte der Hydroformylierung von niederen Olefinen in homogener Phase enthalten. Die kontinuierliche Ausgestaltung eines solchen Verfahrens für die Umsetzung von Olefinen mit 2 bis 20 C-Atomen, insbesondere Ethylen, Propylen, Hexene, Heptene und Octene ist Gegenstand der GB-A-1 312 076. Die Rhodiumkomplexverbindungen sind in beiden Fällen in Wasser unlöslich, überschüssige Liganden liegen nicht vor. Die Trennaufgabe beschränkt sich darauf, das Gemisch in die Rhodiumverbindung und die übrigen Bestandteile, insbesondere Olefine und Aldehyde, zu zerlegen.

Bei der Aufarbeitung wäßriger Lösungen, die als Katalysator verwendete Rhodiumkomplexverbindungen, überschüssige Liganden, deren Abbau- und Umwandlungsprodukte und weitere, die Selektivität des Katalysators mindernde Stoffe enthalten, ergeben sich Probleme, die bei der Behandlung von Koordinationskomplexe der Übergangsmetalle enthaltenden Gemischen mit einer oder mehreren organischen Komponenten nicht auftreten. So entfällt durch die Abtrennung der im Überschuß vorhandenen Liganden ihre stabilisierende Wirkung auf den Rhodiumkomplex. Es ist daher zu erwarten, daß die Komplexverbindung auseinanderbricht und neue Rhodiumkomplexe unter Einschluß von Wasser, das in sehr großem Überschuß vorliegt, gebildet werden. Alternativ dürfte nur ein Bruchteil der überschüssigen Liganden abgetrennt werden, um die ursprüngliche Komplexverbindung zu erhalten. Dann besteht jedoch die Gefahr, daß Katalysatorgifte und andere, möglicherweise schädlich wirkende Stoffe, nur unvollständig entfernt werden und Rhodiumkomplexverbindungen zurückgewonnen werden, die sich erst nach zusätzlichen Reinigungsschritten als Katalysatoren wiederverwenden lassen.

Es bestand daher die Aufgabe ein Verfahren bereitzustellen, das eine möglichst weitgehende Trennung in wäßriger Lösung enthaltender katalytisch aktiver Rhodiumkomplexverbindungen einerseits, von in derselben Lösung vorliegenden überschüssigen, d.h. ungebundenen Komplexliganden, deren Umwandlungs- und Abbauprodukte sowie weitere Verun reinigungen andererseits erlaubt. Dabei sollen Edelmetallverluste so gering wie möglich gehalten werden.

Diese Aufgabe wird gelöst durch ein Verfahren zur Abtrennung von Rhodiumkomplexverbindungen, die als Liganden wasserlösliche organische Phosphine der allgemeinen Formel

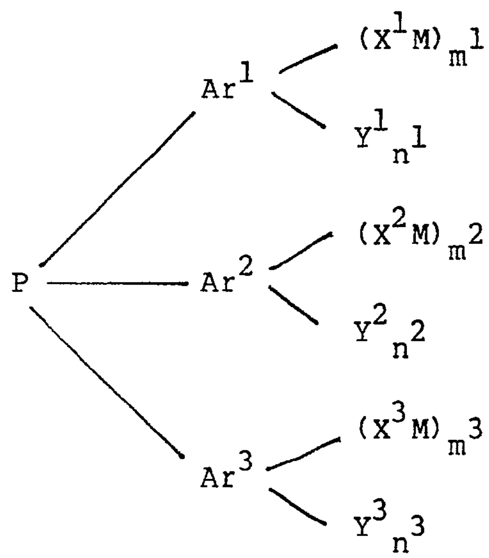

enthalten, in der $Ar^1$, $Ar^2$, $Ar^3$ jeweils eine Phenyl- oder Naphthylgruppe, $Y^1$, $Y^2$, $Y^3$ jeweils eine geradkettige oder verzweigte Alkylgruppe mit jeweils 1 bis 4 C-Atomen, eine Alkoxygruppe mit jeweils 1 bis 4 C-Atomen, ein Halogenatom, die OH-, CN-, $NO_2$- oder $R^1R^2N$-Gruppe bedeuten, in der $R^1$ und $R^2$ für geradkettige oder verzweigte Alkylgruppen mit jeweils 1 bis 4 C-Atomen stehen, $X^1$, $X^2$, $X^3$ jeweils ein Carboxylat-($COO^-$) und/oder Sulfonat-($SO_3$-)Rest ist, $n_1$, $n_2$, $n_3$ gleiche oder verschiedene ganze Zahlen von 0 bis 5 sind, M ein Alkalimetallion, das Äquivalent eines Erdalkalimetall- oder Zinkions oder ein Ammonium- oder quartäres Alkylammoniumion der allgemeinen Formel $N(R^3R^4R^5R^6)^+$, in der $R^3$, $R^4$, $R^5$, $R^6$ jeweils für eine geradkettige oder verzweigte Alkylgruppe mit 1 bis 18 C-Atomen steht, ist und $m^1$, $m^2$, $m^3$ gleiche oder verschiedene ganze Zahlen von 0 bis 3 sind, wobei mindestens eine Zahl $m^1$, $m^2$ oder $m^3$ gleich oder größer als 1 ist, aus wäßrigen Lösungen, in denen außerdem überschüssiger Phosphinligand und gegebenenfalls noch weitere Komponenten gelöst sind. Es ist dadurch gekennzeichnet, daß man die wäßrige Lösung einem Membrantrennverfahren unterwirft.

Bevorzugt werden quartäre Ammoniumionen, in denen drei der Reste $R^3$, $R^4$, $R^5$, $R^6$ jeweils 1 bis 4 und der vierte Rest 1 bis 18 Kohlenstoffatome enthalten.

Die Rhodium-Komplexverbindungen folgen der allgemeinen Formel $HRh(CO)_xL_{4-x}$, wobei L für den wasserlöslichen Phosphinliganden steht und x die Zahlen 1 bis 3 bedeutet.

Die neue Arbeitsweise eignet sich insbesondere zur Abtrennung von Rhodiumkomplexverbindungen aus wäßrigen Lösungen, die als Katalysatorphase bei der Hydroformylierung von Olefinen angewendet werden

und zur Wiederherstellung ihrer ursprünglichen selektiven Wirksamkeit regeneriert werden müssen.

Überraschenderweise ermöglicht das erfindungsgemäße Verfahren, die zumeist in geringer Konzentration vorliegende Rhodiumkomplexverbindung von den übrigen Bestandteilen der Lösung so selektiv abzutrennen, daß sie ohne zusätzliche Reinigungsschritte unmittelbar wieder als Bestandteil des Katalysatorsystems eingesetzt werden können. Dabei auftretende Rhodiumverluste sind vernachlässigbar gering.

Es war nicht zu erwarten, daß nur ganz untergeordnete Edelmetallmengen verloren werden und daß die von den übrigen Bestandteilen der Lösung abgetrennte Rhodiumkomplexverbindung die ursprüngliche Aktivität und selektive Wirkung beibehält. Hierbei ist zu berücksichtigen, daß im Verlauf des Aufarbeitungsprozesses die das Komplexmolekül stabilisierenden Bedingungen verlassen werden. Auf die Wirkung des hohen Wasserüberschusses wurde schon hingewiesen. Das Fehlen starker Komplexliganden im Überschuß sollte zu einem Zerfall des Komplexes gegebenenfalls unter Abscheidung von metallischem Rhodium führen.

Die dem Membrantrennverfahren unterworfenen Lösungen enthalten den Rhodium-Phosphinkomplex und neben Wasser als Hauptbestandteile wasserlösliche organische Phosphine sowie deren Umwandlungs- und Abbauprodukte. Zu ihnen zählen organische Reste enthaltende Phosphinoxide, Phosphinsulfide, Sulfonsäuren, Carbonsäuren. Ferner sind entsprechend ihrer Löslichkeit in Wasser auch Olefine, Aldehyde, Alkohole, Metallcarbonyle, insbesondere Eisencarbonyl, Wasserstoff, Kohlenmonoxid und gegebenenfalls Lösungsvermittler in der Lösung zu finden.

Lösungsvermittler sind Stoffe oder Stoffgemische, die insbesondere bei erhöhten Temperaturen sowohl in der wäßrigen als auch in der organischen Phase löslich sind. Ihre Wirkung besteht vor allem darin, die physikalischen Eigenschaften der Grenzflächen zwischen den beiden flüssigen Phasen zu verändern und dadurch den Übergang der organischen Reaktanten in die Katalysatorlösung zu erleichtern. Beispiele für diese Substanzen sind Salze von Carbonsäuren mit 8 bis 20 Kohlenstoffatomen, Alkylsulfonate, quarternäre Oniumverbindungen, insbesondere Ammoniumsalze, sowie Addukte des Ethylenoxids wie Alkyl-polyethylenglykole.

Es ist zweckmäßig, vor Durchführung des Membrantrennverfahrens die wäßrige Lösung zu filtrieren um grobe Verunreinigungen, die zu einer Verstopfung der Membran führen können, zu entfernen. Weiterhin ist es vorteilhaft, vor der Trennung auch organische Substanzen, vor allem solche, die als Lösungsmittel oder Quellmittel für die Membran wirken, z.B. durch Extraktion, Destillation, Wasserdampfdestillation, aus der Lösung abzutrennen, um eine Schädigung der Membranstruktur auszuschließen.

Unter dem Ausdruck Membrantrennverfahren werden insbesondere die Ultrafiltration und die Hyperfiltration (Reversosmose, umgekehrte Osmose) verstanden.

Ultrafiltration und Hyperfiltration sind selektive Molekulartrennverfahren, bei denen die Membran als Molekularsieb wirkt. Die Membran vermag gelöste Stoffe ausreichender Molekülgröße an der Membrangrenzfläche zurückzuhalten, während kleinere Moleküle die Grenzfläche passieren, so daß eine Separierung der Teilchen nach ihrer Größe erfolgt. Die Trennung wird dadurch bewirkt, daß die großen Moleküle größer sind als die größten Poren der Membran, während die kleinen Moleküle die Membran mehr oder weniger ungehindert durchdringen. Die Trennverfahren sind in der Literatur eingehend abgehandelt, eine zusammenfassende Beschreibung findet sich z.B. in Ullmanns Encyklopädie der technischen Chemie, 4. Auflage (1978), Bd. 16, S. 515 ff.

Nach der erfindungsgemäßen Arbeitsweise führt man die zu trennende wäßrige Lösung einer Seite der Membran unter einem Druck zu, der größer ist als der Druck auf der gegenüberliegenden Seite der Membran. Der Druckunterschied muß dabei größer sein als der osmotische Druck des Systems.

Als Membranmaterialien haben sich z.B. Cellulose, Celluloseacetat, Polyamide, Polyimide, Polyolefine, Polyvinylalkohole, Polyacrylnitrile, Polysulfone und sulfonierte Polysulfone bewährt. Besonders geeignet sind Celluloseacetat, Polyimide, Polysulfone und sulfonierte Polysulfone. Bevorzugt werden Celluloseacetat und Polysulfone.

Die Membranen sollen so dünn wie möglich sein. Gefordert wird lediglich, daß sie den angewandten äußeren Bedingungen des Trennverfahrens, insbesondere dem Druck standhalten. Vorzugsweise setzt man Membranen ein, deren Dicke 50 bis 200 $\mu$m beträgt.

Erwünscht ist eine möglichst hohe Lebensdauer der Membranen, um die Wirtschaftlichkeit des Verfahrens sicherzustellen. Membranen aus Celluloseacetat erfüllen diese Voraussetzungen in besonderem Maße. Sie halten bei kontinuierlicher Arbeitsweise über mehr als 200 Tage wenigstens 98 % der Rhodiumkomplexverbindung zurück, wenn 80 % der in der Lösung vorhandenen Salze als Permeat abgetrennt werden.

Zur Trennung des eingesetzten Substrats arbeitet man mit strömungsführenden Geräten, sogenannten Modulen. Sie haben unterschiedliche, für die jeweilige Anwendung geeignete Gestalt und können, z.B. in Form von Rohren, Platten oder Spiralen eingesetzt werden. In den Modulen sind die Membranen als Flach-

oder Schlauchfolien angeordnet.

Die Arbeitsbedingungen bei dem erfindungsgemäßen Trennverfahren hängen wesentlich von der Membran und der Zusammensetzung der Lösung, die getrennt werden soll, ab. Die Hauptvariablen des Verfahrens sind der angewandte Druck sowie Temperatur und Konzentration der Lösung.

Wie bereits gesagt, muß der Druck größer sein als der osmotische Druck des Systems. Er wird weiterhin vom Membranmaterial und der Modulbauweise beeinflußt. Drücke von 0,6 bis 6,0 MPa und insbesondere von 1,2 bis 2,0 MPa haben sich bewährt.

Die Lösung wird in das Trennverfahren mit einer Temperatur von 10 bis 80 °C, vorzugsweise von 20 bis 35 °C eingesetzt. Es hat sich als zweckmässig erwiesen, daß ihre Salzkonzentration (d.h. die Gesamtheit aller als Salze vorliegenden Verbindungen der Trockensubstanzgehalt) 1,0 bis 20 Gew.-% vorzugsweise 3 bis 16 Gew.-%, und insbesondere 5 bis 12 Gew.-% (jeweils bezogen auf die Lösung) beträgt. Rhodium soll in der Löung in einer Konzentration von 10 bis 1000 ppm, insbesondere 50 bis 500 ppm vorliegen.

Da die Lösungen entsprechend ihrer Verwendung als Katalysator üblicherweise 10 bis 50 Gew.-% Salze und 100 bis 2000 ppm Rhodium (jeweils bezogen auf die Lösung) enthalten, sind die für die Trennung geeigneten Konzentrationen gegebenenfalls durch Verdünnen der einzusetzenden Lösung mit entionisiertem Wasser einzustellen.

An den pH-Wert der aufzuarbeitenden Lösung werden keine besonderen Anforderungen gestellt. Im allgemeinen genügt es, innerhalb eines pH-Bereiches von 3 bis 10, vorzugsweise 4 bis 9, und insbesondere 5 bis 8, zu arbeiten. Bei Verwendung einer Celluloseacetatmembran empfiehlt es sich, pH-Werte von 3,5 bis 6, insbesondere von 4 bis 5, einzustellen.

Das neue Verfahren kann absatzweise durchgeführt werden. In diesem Fall trennt man die zu regenerierende Katalysatorlösung von den übrigen Reaktionsteilnehmern ab, arbeitet sie erfindungsgemäß auf und setzt sie erneut ein.

Besonders bewährt hat es sich jedoch, kontinuierlich zu arbeiten. Bei dieser Verfahrensvariante wird ein Anteil der Katalysatorlösung kontinuierlich aus dem Prozeß, in dem sie eingesetzt ist, abgezogen und dem Membrantrennverfahren unterworfen. Die Menge der abgezogenen Katalysatorlösung richtet sich insbesondere nach dem Ausmaß des Abbaus und der Umwandlung des Liganden. Die von der Membran zurückgehaltene, die Rhodiumkomplexverbindung enthaltende Lösung (Retentat) wird dem Prozeß wieder zugeleitet, gegebenenfalls nach Zusatz von wasserlöslichem organischem Phosphin, um das gewünschte Rh/P-Verhältnis in der Katalysatorlösung aufrechtzuerhalten. In der Lösung, die die Membran passiert hat (Permeat), befinden sich die Umwandlungsprodukte der Komplexliganden, die komplexbildenden organischen Phosphinsulfonate bzw.-carboxylate, gegebenenfalls Verunreinigungen sowie in Spuren Rhodium. Das Permeat wird zur Rückgewinnung von Rhodium und Phosphin, aufgearbeitet. Hierzu eignet sich z.B., das als Stand der Technik weiter oben gewürdigte Verfahren der DE 32 35 029 A1.

In Figur 1 ist eine Anordnung zur diskontinuierlichen Durchführung des erfindungsgemäßen Verfahrens dargestellt.

Die eine Rhodiumkomplexverbindung enthaltende Lösung wird über eine Leitung 2 einem Behälter 1 zugeführt. Durch Zusatz von entionisiertem Wasser über eine Leitung 8 kann die gewünschte Salz- und Rhodiumkonzentration eingestellt werden. Uber eine Leitung 3 gelangt die Lösung zu einer Pumpe 4 und wird hier auf den erforderlichen Arbeitsdruck gebracht. In einem Modul 5, das die Membran enthält, erfolgt die Trennung der Lösung in Permeat und Retentat. Das Permeat wird über eine Leitung 7 der Aufarbeitung zugeführt, das Retentat kehrt über eine Leitung 6 in den Behälter 1 zurück.

Fig. 2 zeigt die kontinuierliche Ausgestaltung der neuen Arbeitsweise zur Regenerierung einer ausgebrauchten Katalysatorlösung. Die Katalysatorlösung wird über eine Leitung 1, gegebenenfalls nach Verdünnung mit entionisiertem Wasser, das über eine Leitung 2 eingespeist wird, einem die Membran enthaltenen Modul 3 zugeführt. Das Permeat wird über eine Leitung 4 abgezogen. Eine Teilmenge des Retentats strömt durch eine Leitung 5 wieder zum Modul 3, eine andere Teilmenge wird über eine Leitung 6 in den Reaktor eingeleitet.

In den folgenden Beispielen wird das erfindungsgemäße Verfahren näher erläutert. Der Ausdruck "Salz" wird für Gemische verwendet, die im wesentlichen die Alkalisalze der Triphenylphosphin-, Triphenylphosphinoxid- und Triphenylphosphinsulfidmonosulfonsäure, -disulfonsäure und -trisulfonsäure und Alkalisalze aromatischer Sulfonsäuren enthalten.

Beispiel 1

Die Versuche a bis g werden in der in Fig. 1 dargestellten Apparatur durchgeführt. Das abgetrennte Permeat wird jedoch nicht einer Aufarbeitung zugeleitet, sondern mit dem Retentat vereinigt und in den Behälter 1 zurückgeführt. Ziel der Versuche ist die Ermittlung der Trennleistung einer im Gleichgewicht

befindlichen Membran bei Einsatz gleicher Volumina von Lösungen unterschiedlicher Konzentrationen und bei Anwendung unterschiedlicher Drücke.

Die zur Durchführung der Versuche verwendete Membran (ein Produkt der Firma Millipore, Handelsbezeichnung PCAC) besteht aus Celluloseacetat. Sie hat eine Fläche von 4,2 $m^2$ und wird als Wickelmodul eingesetzt. Ihre molekulare Trenngrenze ist 1000.

Die aufzuarbeitenden wäßrigen Lösungen enthalten 7,05, 8,90 bzw. 10,84 Gew.-% Salze und 88, 111 bzw. 136 ppm Rhodium jeweils bezogen auf die Lösung. Sie haben eine Temperatur von 27 bis 30° C. Je Stunde leitet man der Membran 700 l Lösung zu.

Die Versuchsbedingungen und die Versuchsergebnisse sind in Tabelle 1 zusammengestellt.

Tabelle 1: Beispiel 1

Versuchsbedingungen:

| Einsatz-lösung | Versuch | Druck (MPa) | Permeat-fluß (kg/h) | Rhodim im Permeat (ppm) | abge-trenn-tes Rh (mg/h) | Salzgehalt im Permeat Gew.-% | abge-trenntes Salz in kg/h | abge-trenntes Salz kg/mg Rh |
|---|---|---|---|---|---|---|---|---|
| 7,05 Gew.-% Salz | a | 1,2 | 34,02 | 0,21 | 7,14 | 2,29 | 0,779 | 0,1091 |
| 88 ppm Rh $d_{20}$ = 1,038 | b | 1,4 | 43,05 | 0,20 | 8,61 | 2,19 | 0,943 | 0,1095 |
| 8,90 Gew.-% Salz | c | 1,0 | 15,96 | 0,47 | 7,50 | 3,77 | 0,602 | 0,0803 |
| 111 ppm Rh $d_{20}$ = 1,048 | d | 1,2 | 23,27 | 0,42 | 9,77 | 3,35 | 0,779 | 0,0797 |
| | e | 1,4 | 30,58 | 0,49 | 14,98 | 3,17 | 0,969 | 0,0647 |
| 10,84 Gew.-% Salz | f | 1,2 | 12,94 | 0,59 | 7,63 | 4,65 | 0,601 | 0,0783 |
| 136 ppm Rh $d_{20}$ = 1,058 | g | 1,4 | 18,10 | 0,57 | 10,32 | 4,25 | 0,769 | 0,0745 |

Beispiel 2

In der in Figur 1 dargestellten Apparatur werden aus Behälter 1 84 l (entsprechend 88,5 kg) einer wäßrigen Lösung, die 8,9 Gew.-%Salz und 111 ppm Rhodium (jeweils bezogen auf die Lösung) enthält, in einer Menge von 660 l/h einer Celluloseacetat-Membran Wickelmodul; Fläche: 4,2 $m^2$; ein Produkt der Fa. Milipore) zugeleitet. Der Druck beträgt 1,2 bis 1,6 MPa, die Temperatur der Lösung 30°C. Man erhalt 18

bis 25 l Permeat/h. Die Salzkonzentration im Permeat beträgt 3 bis 3,7 Gew.-%, die Rhodiumkonzentration <1 ppm (jeweils bezogen auf Permeatlösung).

Das Retentat wird im Kreis gefahren und der Membran wieder zugeführt; durch Zugabe von entionisiertem Wasser wird im Retentat die ursprüngliche Salzkonzentration von 8,9 Gew.-% aufrechterhalten.

Setzt man die Salzabtrennung soweit fort, daß nur noch 18 % der in der Ausgangslösung vorliegenden Salzmenge im Retentat enthalten sind, finden sich im Retentat ~640 ppm Rh, im Permeat ~3 ppm Rh.

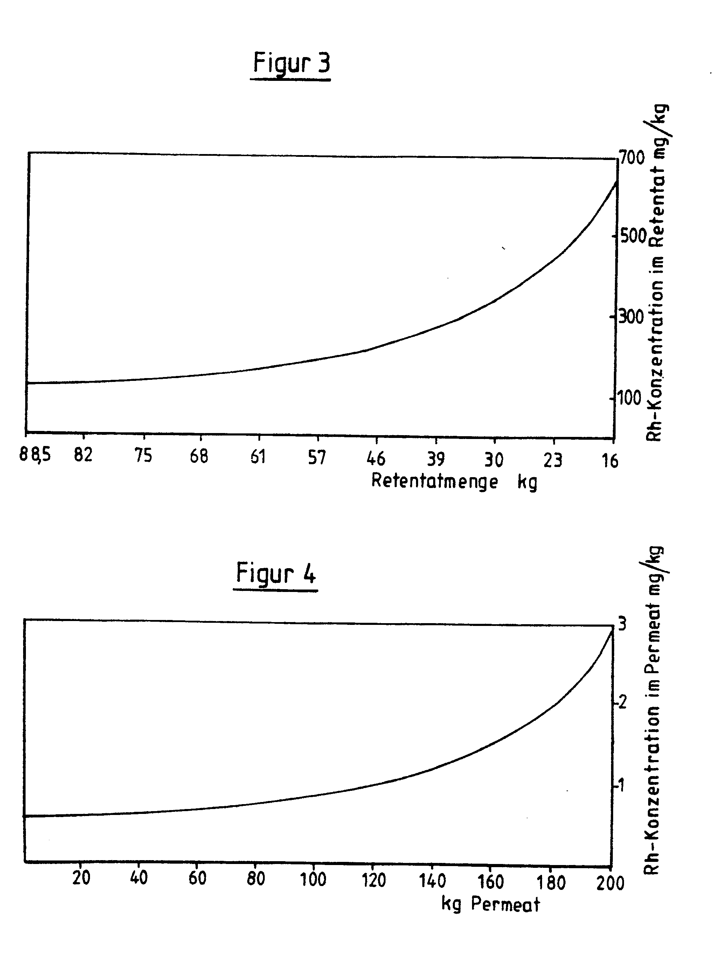

In Figur 3 ist die Abhängigkeit der Rhodiumkonzentration von der Menge Retentat und in Figur 4 die Abhängigkeit der Rhodiumkonzentration von der jeweils abgetrennten Menge Permeat wiedergegeben.

Wie aus dem Kurvenverlauf in den beiden Diagrammen hervorgeht, hält die Membran unabhängig von der Rhodiumkonzentration im Retentat stets mehr als 98 % des im Retentat enthaltenen Rhodiums zurück.

Das Verhältnis Rh zu P(III) (in g-Atom je mol) beträgt zu Beginn des Abtrennungsvorganges 1 : 39 und liegt am Ende der Trennung bei 1 : 7.

Die Erhaltung der ursprünglichen Aktivität und selektiven Wirkung des Katalysatorsystems durch Behandlung nach dem erfindungsgemäßen Verfahren zeigt das folgende Beispiel.

Beispiel 3

In einem Rührkessel, der 45 1 wäßrige Katalysatorlösung (500 Gew.-ppm Rh, bezogen auf die Lösung; je g-Atom Rh 80 mol Triphenylphosphintrisulfonat) enthält, werden bei 120 °C und einem Druck von 5,0 MPa stündlich 5,5 kg Propylen und 6 $Nm^3$ Synthesegas ($CO:H_2$ = 1;1) geleitet. Man erhält je Stunde 8 kg Butyraldehyd mit einem n/i-Verhältnis von 95 : 5.

Mit zunehmender Laufzeit verringert sich das P(III) : Rh-Verhältnis durch Bildung von P(V)-Verbindungen mit der Folge, daß zu Lasten des n-Aldehyds vermehrt i-Aldehyd gebildet wird. Um das P(III) : Rh-Verhältnis konstant zu halten, werden dem System je Tag 1,5 bis 3 1 Katalysatorlösung entzogen und nach dem erfindungsgemäßen Verfahren über eine Membran getrennt. Man erhält 5 bis 10 1 Retentatlösung. Diese Lösung - ergänzt um den aus dem Permeat zurückgewonnenen Anteil an P(III)-Salzen (0,1 - 0,15 kg/d) - wird dem Reaktionssystem wieder zugeführt. Die notwendige Wasserergänzung wird um die zurückgeführte Menge Retentat reduziert.

Das Reaktionssystem kann unter den angegebenen Bedingungen monatelang ohne Aktivitäts- bzw. Selektivitätseinbußen kontinuierlich betrieben werden.

**Patentansprüche**

1. Verfahren zur Abtrennung von Rhodiumkomplexverbindungen, die als Liganden wasserlösliche organische Phosphine der allgemeinen Formel

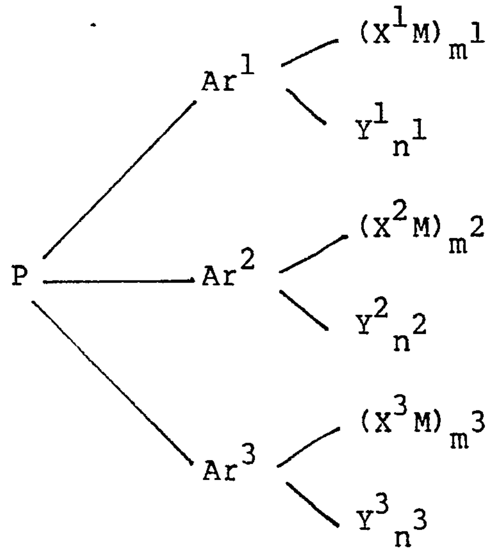

enthalten, in der $Ar^1$, $Ar^2$, $Ar^3$ jeweils eine Phenyl- oder Naphthylgruppe, $Y^1$, $Y^2$, $Y^3$ jeweils eine geradkettige oder verzweigte Alkylgruppe mit jeweils 1 bis 4 C-Atomen, eine Alkoxygruppe mit jeweils

1 bis 4 C-Atomen, ein Halogenatom, die OH-, CN-, $NO_2$- oder $R^1R^2$ N-Gruppe bedeuten, in der $R^1$ und $R^2$ für geradkettige oder verzweigte Alkylgruppen mit jeweils 1 bis 4 C-Atomen stehen, $X^1$, $X^2$, $X^3$ jeweils ein Carboxylat-(COO) und/oder Sulfonat-($SO_3$-)Rest ist, $n_1$` $n_2$ $n_3$ gleiche oder verschiedene ganze Zahlen von 0 bis 5 sind, M ein Alkalimetallion, das Äquivalent eines Erdalkalimetall- oder Zinkions oder ein Ammonium- oder quartäres Alkylammoniumion der allgemeinen Formel $N(R^3R^4R^5R^6)^+$, in der $R^3$, $R^4$, $R^5$, $R^6$ jeweils für eine geradkettige oder verzweigte Alkylgruppe mit 1 bis 18 C-Atomen steht, ist und $m^1$, $m^2$, $m^3$ gleiche oder verschiedene ganze Zahlen von 0 bis 3 sind, wobei mindestens eine Zahl $m^1$, $m^2$ oder $m^3$ gleich oder größer als 1 ist, aus wäßrigen Lösungen¯ in denen außerdem überschüssiger Phosphinligand und gegebenenfalls noch weitere Komponenten gelöst sind, dadurch gekennzeichnet, daß man die wäßrige Lösung einem Membrantrennverfahren unterwirft.

**2.** Verfahren nach Anspruch 1, dadurch gekennzeichnet, daß vor Durchführung des Membrantrennverfahrens die wäßrige Lösung filtriert wird.

**3.** Verfahren nach einem oder mehreren der Ansprüche 1 und 2, dadurch gekennzeichent, daß vor Durchführung des Membrantrennverfahrens flüchtige organische Substanzen aus der wäßrigen Lösung abgetrennt werden.

**4.** Verfahren nach einem oder mehreren der Ansprüche 1 bis 3, dadurch gekennzeichnet daß als Membrantrennverfahren die Ultrafiltration oder die Hyperfiltration angewandt wird.

**5.** Verfahren nach einem oder mehreren der Ansprüche 1 bis 4, dadurch gekennzeichnet, daß als Membranmaterial Celluloseacetat verwendet wird.

**6.** Verfahren nach einem oder mehreren der Ansprüche 1 bis 5, dadurch gekennzeichnet daß Drucke von 0,6 bis 6,0 MPa angewandt werden.

**7.** Verfahren nach einem oder mehreren der Ansprüche 1 bis 6, dadurch gekennzeichnet daß der Trockensubstanzgehalt der Lösung vor Trennung 1,0 bis 20 Gew.-% bezogen auf die Lösung, beträgt.

**8.** Verfahren nach einem oder mehreren der Ansprüche 1 bis 7, dadurch gekennzeichnet, daß der pH-Wert der Lösung 3 bis 10, insbesondere 4 bis 9, bevorzugt 5 bis 8 beträgt.

## Claims

**1.** A process for separating rhodium complex compounds containing as ligands water-soluble organic phosphines from aqueous solutions in which other components can be dissolved in addition to the excess phosphine ligand, said water-soluble organic phosphines having the general formula

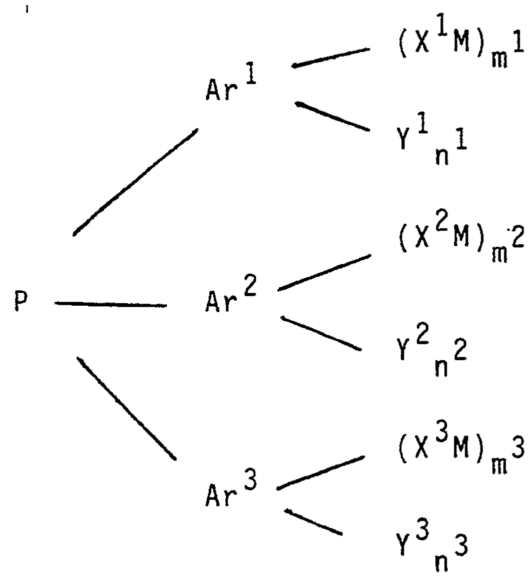

where $Ar^1$, $Ar^2$ and $Ar^3$ are each a phenyl or naphthyl group, $Y^1$, $Y^2$ and $Y^3$ each denote a straight-chain or branched alkyl group having 1 to 4 carbon atoms, an alkoxy group having 1 to 4 carbon atoms, a halogen atom, the OH, GM, $NO_2$ or $R^1R^2N$ group where $R^1$ and $R^2$ stand for straight-chain or branched alkyl groups each having 1 to 4 carbon atoms, $x^1$, $X^2$ and $X^3$ are each a carboxylate ($COO^-$) and/or sulfonate ($SO_3$) radical, $n_1$, $n_2$ and $n_3$ are each the same or different integers from 0 to 5, M is an alkali metal ion, the equivalent of an alkaline earth metal or zinc ion or an ammonium or quaternary alkylammonium ion of the general formula $N(R^3R^4R^5R^6)+$, where $R^3$, $R^4$, $R^5$ and $R^6$ each stand for a straight-chain or branched alkyl group having 1 to 18 carbon atoms, and $m^1$, $m^2$ and $m^3$ are the same or different integers from 0 to 3, at least one number $m^1$, $m^2$ or $m^3$ being equal to or greater than 1, characterised in that the aqueous solution is subjected to a membrane separation process.

2. A process according to claim 1, characterised in that the aqueous solution is filtered before being subjected to the membrane separation process.

3. A process according to one or both of the claims 1 and 2, characterised in that volatile organic substances are removed from the aqueous solution before the membrane separation process is performed.

4. A process according to one or more of the claims 1 to 3, characterised in that ultrafiltration or hyperfiltration is employed as the membrane separation process.

5. A process according to one or more of the claims 1 to 4, characterised in that cellulose acetate is used as the membrane material

6. A process according to one or more of the claims 1 to 5, characterised in that pressures of 0.6 to 6.0 MPa are applied.

7. A process according to one or more of the claims 1 to 6, characterised in that the dry substance content of the solution before separation is 1.0 to 20% by weight, related to the solution.

8. A process according to one or more of the claims 1 to 7, characterised in that the pH value of the solution is 3 to 10, in particular 4 to 9, preferably 5 to 8.

## Revendications

1. Procédé pour la séparation de composés complexes de rhodium qui contiennent comme ligands des phosphines organiques solubles dans l'eau de formule générale

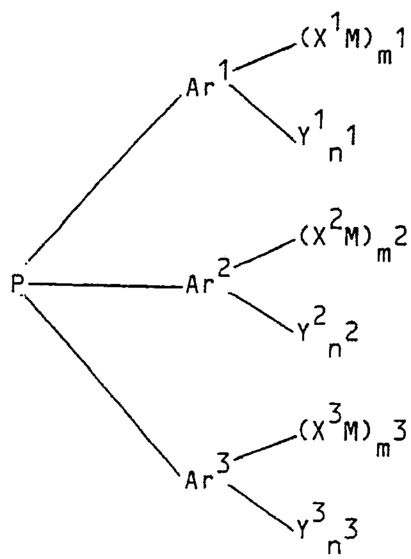

dans laquelle $Ar^1$, $Ar^2$ et $Ar^3$ représentent chacun un groupe phényle ou naphtyle, $Y^1$, $Y^2$ et $Y^3$ représentent chacun un groupe alkyle à chaîne droite ou ramifiée en $C_1$-$C_4$, un groupe alcoxy en $C_1$-$C_4$, un atome d'halogène, le groupe 0H, CN, $NO_2$ ou $R^1R^2N$, dans lequel $R^1$ et $R^2$ représentent des groupes alkyles chaque fois à chaîne droite ou ramifiée en $C_1$ -$C_4$, $X^1$, $X^2$ et $X^3$ représentent chacun un reste carboxylate (C00) et/ou un reste sulfonate ($SO_3$), $n_1$, $n_2$ et $n_3$ sont des nombres entiers identiques ou différents de 0 à 5, M est un ion de métal alcalin, l'équivalent d'un ion de métal alcalino-terreux ou d'un ion zinc ou un ion ammonium ou alkylammonium quaternaire de formule générale $N-(R^3R^4R^5R^6)^+$, dans laquelle $R^3$, $R^4$, $R^5$ et $R^6$ représentent chacun un groupe alkyle à chaîne droite ou ramifiée en $C_1$-$C_{18}$, et $m^1$ , $m^2$ et $m^3$ sont des nombres entiers, identiques ou différents, de 0 à 3, un nombre $m^1$ , $m^2$ ou $m^3$ au moins étant égal ou supérieur à 1, de solutions aqueuses dans lesquelles sont dissous en outre le ligand phosphine en excès et éventuellement encore d'autres composants, caractérisé en ce que l'on soumet la solution aqueuse à un procédé de séparation sur membrane.

2. Procédé selon la revendication 1, caractérisé en ce que l'on filtre la solution aqueuse avant la mise en oeuvre du procédé de séparation sur membrane.

3. Procédé selon une ou plusieurs des revendications 1 et 2, caractérisé en ce que l'on sépare les substances organiques volatiles de la solution aqueuse avant la séparation.

4. Procédé selon une ou plusieurs des revendications 1 à 3, caractérisé en ce que l'on applique comme procédé de séparation sur membrane l'ultrafiltration ou l'hyperfiltration.

5. Procédé selon une ou plusieurs des revendications 1 à 4, caractérisé en ce que l'on utilise comme matière de membrane l'acétate de cellulose.

6. Procédé selon une ou plusieurs des revendications 1 à 5, caractérisé en ce que l'on applique des pressions de 0,6 à 6,0 MPa.

7. Procédé selon une ou plusieurs des revendications 1 à 6, caractérisé en ce que la teneur en matières sèches de la solution avant la séparation est de 1,0 à 20 % en poids, par rapport à la solution.

8. Procédé selon une ou plusieurs des revendications 1 à 7, caractérisé en ce que le pH de la solution est de 3 à 10, en particulier 4 à 9, de préférence 5 à 8.

Figur 1

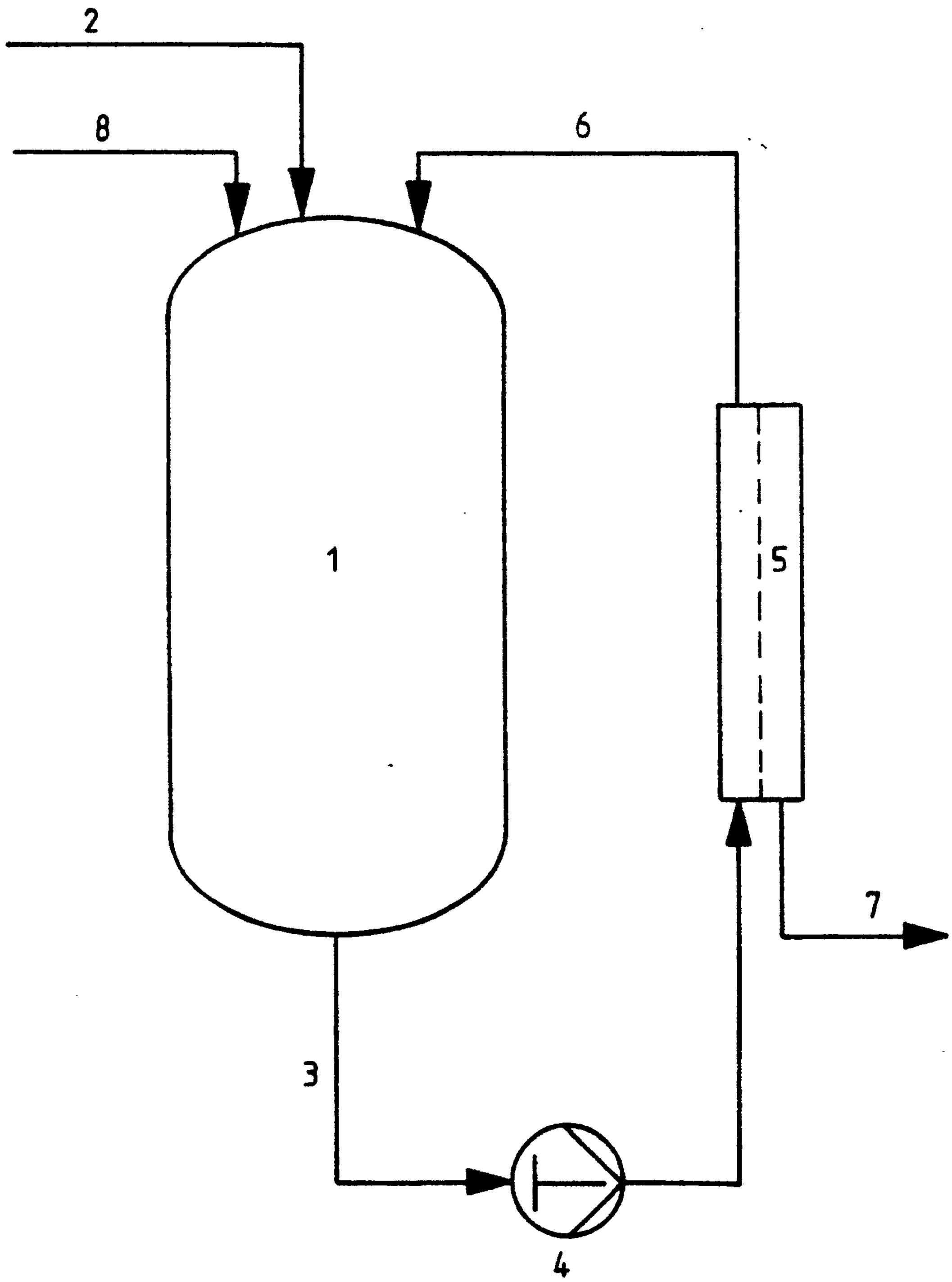

Figur 2

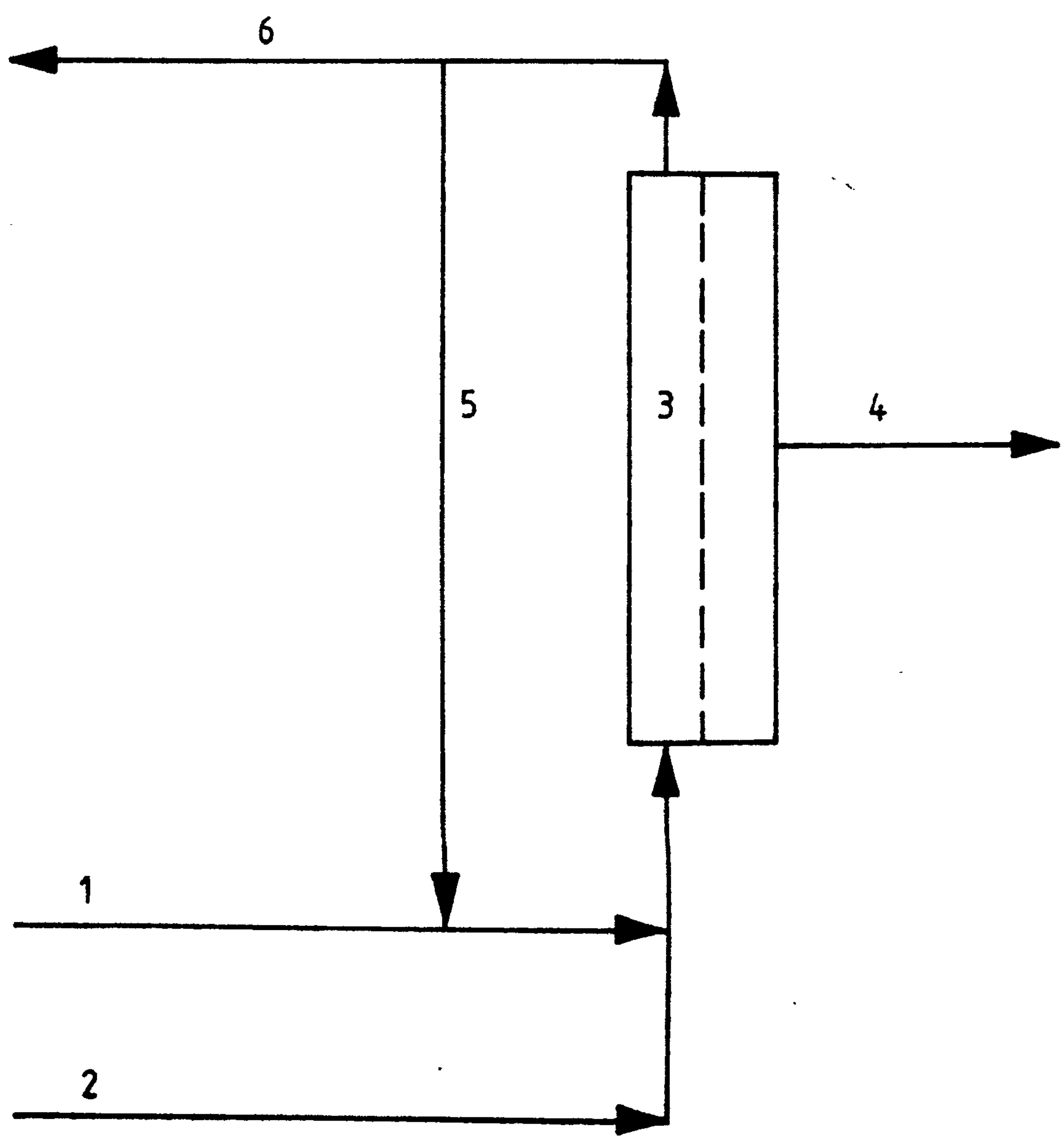

Figur 3

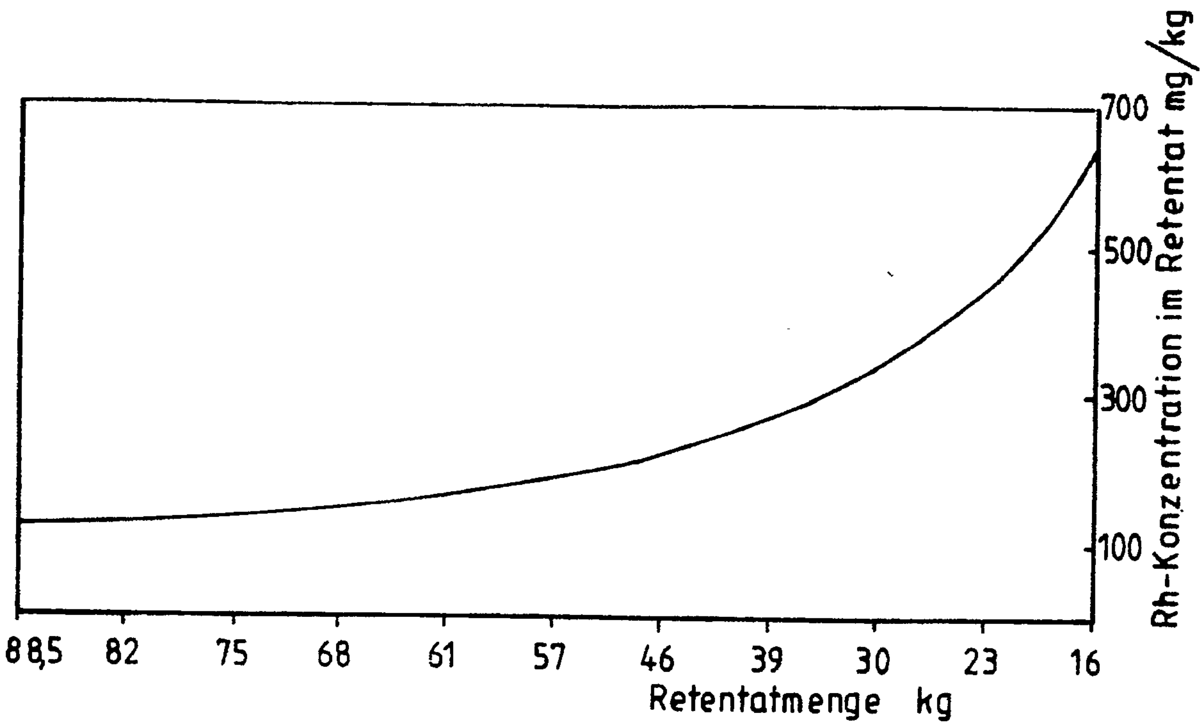


Figur 4